(19) Europäisches Patentamt
European Patent Office
Office européen des brevets

(11) Publication number: **0 173 531**
**A1**

(12) **EUROPEAN PATENT APPLICATION**

(21) Application number: **85305885.7**

(22) Date of filing: **19.08.85**

(51) Int. Cl.[4]: **C 07 D 319/06**, A 01 N 43/32
// C10M173/00, C10N40:20

(30) Priority: **29.08.84 GB 8421788**

(43) Date of publication of application: **05.03.86**
**Bulletin 86/10**

(84) Designated Contracting States: **AT BE CH DE FR GB IT LI LU NL SE**

(71) Applicant: **IMPERIAL CHEMICAL INDUSTRIES PLC, Imperial Chemical House Millbank, London SW1P 3JF (GB)**

(72) Inventor: **Tury, Bernard, 8 Spring Vale, Prestwich Manchester M25 8SE (GB)**
Inventor: **Wilton, Jeremy Simon, 42 Edenfield Road, Prestwich Manchester M25 8EE (GB)**

(74) Representative: **Colens, Alain M.G.M. et al, Imperial Chemical Industries PLC Legal Department: Patents P.O. Box 6 Bessemer Road, Welwyn Garden City Hertfordshire AL7 1HD (GB)**

(54) **5-Bromo-2-Bromomethyl-5-nitrodioxan and its use as a biocide.**

(57) The invention relates to a new compound, 5-bromo-2-bromomethyl-5-nitrodioxan and its use as a biocide, more particularly in industrial aqueous media such as metal-working fluids.

EP 0 173 531 A1

# 5 - BROMO - 2 - BROMOMETHYL - 5 - NITRODIOXAN AND ITS USE AS A BIOCIDE

This invention relates to a new chemical compound and to its use in the protection of media against infection by micro-organisms.

It is known from United Kingdom Patent Specification No.1250725 that 5-bromo-5-nitro-1,3-dioxan has anti-microbial activity. It has now been found that 5-bromo-2-bromomethyl-5-nitro-1,3-dioxan possesses excellent anti-microbial activity being especially effective against the micro-organisms prevalent in industrial aqueous media.

Accordingly, the invention provides the compound 5-bromo-2-bromomethyl-5-nitro-1,3-dioxan.

The 5-bromo-2-bromomethyl-5-nitro-1,3-dioxan may be prepared by reacting 2-bromo-2-nitro-1,3-propanediol with a dialkyl acetal of bromoacetaldehyde in the presence of an acid catalyst, for example polyphosphoric acid.

The invention also provides a method for the protection of a medium or substrate against attack by micro-organisms which comprises the incorporation therein of an effective amount of 5-bromo-2-bromomethyl-5-nitro-1,3-dioxan. hereafter sometimes referred as BBMND or SC100424. The term "micro-organism" used herein includes bacteria, fungi and algae and the compound of the invention is especially useful for the preservation of aqueous media from attack by such micro-organisms. Examples of such media include cooling waters, paper mill liquors, aqueous emulsions, water-based paints and metal-working fluids. The compound may also have utility in oil industry applications where bacterial spoilage is a problem such as in drilling muds and secondary oil recovery fluids.

The quantity of the 5-bromo-2-bromomethyl-5-nitro-1,3-dioxan required depends on the application but is preferably from 1 part per million (ppm) to 10,000 ppm and more preferably from 10 ppm to 1000 ppm.

The invention is illustrated but not limited by the following Examples.

Example 1

Preparation of 5-bromo-2-bromomethyl-5-nitro-1,3-dioxan (BBMND)

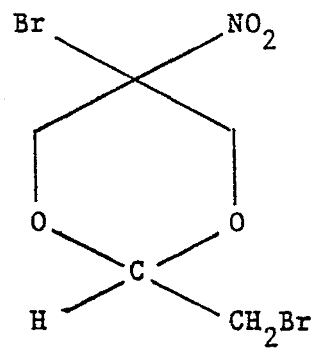

A mixture of 38.0g bromoacetaldehyde diethyl acetal, 39.0g 2-bromo-2-nitro-1,3-propanediol and 0.25g concentrated hydrochloric acid, as catalyst, was heated to 100°C with constant stirring in a 250ml round bottom multineck flask, fitted with a Vigreux column. Within about 15 minutes 8 parts of ethanol had distilled from the flask and distillation became much slower. After 1 hour the Vigreux column was removed and a further 4g ethanol were removed by normal distillation. The temperature was then raised to 120°C for 1 hour to ensure the removal of all ethanol.

On cooling to ambient the dark reaction mass obtained was transferred to a glass tube oven for distillation. The main fraction (10.0g) distilled at 145-150°C with 0.2mm Hg.

Elemental Analysis

| | C | H | N | Br |
|---|---|---|---|---|
| % Calculated: | 20.3 | 2.4 | 4.8 | 54.2 |
| % Found: | 20.9 | 2.7 | 5.2 | 47.4 |

IR Analysis

| Result | Comment | |
|---|---|---|
| Over 3000$cm^{-1}$ | No absorption: | i.e. No OH groups |
| At 2890$cm^{-1}$ | Weak absorption: | Aliphatic C-H group |
| At 1555$cm^{-1}$ | Strong absorption: | Aliphatic Nitro group. |

Example 2

Invitro activity of BBMND

The product of Example 1 was prepared as a 1% solution in dimethyl formamide and diluted when necessary in deionised water.

The solution was added to 50ml volumes of a natural filter-sterilized hard water (pH 8.5) to give active ingredient levels in the 200-3.1 ppm ranges as shown Table 1, where were stored at 50°C for 48 hours. The variants were then cooled to 30°C, transferred to sterile conical flasks, and inoculated with 1ml of an 18-hour nutrient broth culture of Pseudomonas fluorescens NCIB 9046.

The variants were incubated at 30°C in an orbital shaker (100 rev/min) and 1ml samples were removed after contact periods of 2, 6 and 24 hours for viable count determinations by the spread plate technique.

A control contained no BBMND but was otherwise identical to the solutions being assessed.

Table 1

| Biocide | Level (ppm active ingredient) | Survivors (cells/ml) after contact periods of | | |
|---|---|---|---|---|
| | | 2 hours | 6 hours | 24 hours |
| SC100424 | 200 | <100 | <100 | <100 |
| | 50 | <100 | <100 | <100 |
| | 12.5 | $>3.0x10^5$ | $1.87x10^4$ | $4.0x10^2$ |
| | 3.1 | $>3.0x10^5$ | $>3.0x10^5$ | $>3.0x10^5$ |
| Control | | $3.0x10^6$ | $1.7x10^7$ | $1.1x10^7$ |

Example 3

Bactericidal activity of BBMND and Bronopol against Pseudomonas Fluorescens in the presence of slimes

The chemicals were added to 40ml volumes of sterile pH 8.5 water to give active ingredient levels in the 200-3.1 ppm range (on subsequent addition of 10ml of inoculum) and stored at 50°C for 48 hours. Variants were then cooled to 30°C, transferred to 100ml

sterile conical flasks, and inoculated. The inoculum consisted of 10ml of a suspension in deionised water of a growth of Ps.fluorescens NCIB 9406 from a sucrose nutrient agar slant (3 days at 25°C), which provided conditions for significant slime formation.

Variants were incubated at 30°C in an orbital shaker (100 rev/min) and survivors were determined after contact periods of 2, 6 and 24 hours. A control contained no biocide but was otherwise identical to the solutions being assessed.

Table 2

| Biocide | Level (ppm active ingredient) | Survivors (cells/ml) after contact periods of 2 hours | 6 hours | 24 hours |
|---|---|---|---|---|
| SC100424 | 200 | $1.2x10^3$ | $5.0x10^3$ | <100 |
| | 50 | $>3.0x10^5$ | $2.8x10^3$ | <100 |
| | 12.5 | $>3.0x10^5$ | $>3.0x10^5$ | $>3.0x10^5$ |
| | 3.1 | $>3.0x10^5$ | $>3.0x10^5$ | $>3.0x10^5$ |
| Bronopol | 200 | $>3.0x10^5$ | $7.0x10^5$ | <100 |
| | 50 | $>3.0x10^5$ | $>3.0x10^5$ | $6.0x10^4$ |
| | 12.5 | $>3.0x10^5$ | $>3.0x10^5$ | $>3.0x10^5$ |
| | 3.1 | $>3.0x10^5$ | $>3.0x10^5$ | $>3.0x10^5$ |
| Control | | $6.6x10^8$ | $1.8x10^8$ | $9.0x10^8$ |

The results indicate that after 2-, 6- and 24-hour contact periods BBMND is more active than Bronopol. Thus, BBMND appears to be more effective than Bronopol in penetrating bacterial slime.

Example 4

Antialgal activity of BBMND and Bronopol (trade name, 2-bromo-2 nitropropane-1,3-diol)

The chemicals were added to 50ml volumes of natural hard water (pH 8.5), containing 0.1g/litre of added ammonium nitrate to give levels of 25-0.78 ppm active ingredient. After an 18 hour equilibration period at room temperature the variants were inoculated with 1ml of a mixed culture of Chlorella vulgaris,

Pseudococcomyxn adhaerans and Anabaena sp. The variants were incubated at 18°C under artificial illumination and examined for visible algal growth after 2- and 4-week contact periods. A control contained no biocide but was otherwise identical to the solutions being assessed.

Table 3

| Chemical | Level (ppm active ingredient) | Visible Algal Growth after 2 weeks | 4 weeks |
|---|---|---|---|
| SC100424 | 25 | - | - |
| | 12.5 | - | - |
| | 6.25 | - | - |
| | 3.125 | - | - |
| | 1.56 | + | + |
| | 0.78 | + | ++ |
| Bronopol | 25 | - | - |
| | 12.5 | - | - |
| | 6.25 | - | + |
| | 3.125 | + | ++ |
| | 1.56 | ++ | ++ |
| | 0.78 | ++ | ++ |
| Control | | ++ | ++ |

Key: ++ visible growth comparable to that of the control.
+ visible growth sparce/markedly less than that of control.
- no visible growth.

The results indicate that BBMND has higher antifungal activity than Bronopol.

Example 5

Antibacterial activity of BBMND and 1,2-benzisothiazolin-3-one (BIT) in Metal Working Fluid

BBMND as a 1% solution in dimethyl formamide, was added to 100ml volumes of a 5% oil-in-water emulsion, prepared from a Burmah

Castrol emulsifiable oil, to give levels in the 200-25 ppm range. 1,2-Benzisothiazolin-3-one (BIT) as a solution was used as the standard.

The variants were transferred to an orbital shaker (100 rev/min; 30°C) and inoculated once weekly for 3 weeks with 1ml of a 1 in 10,000 dilution of an 18 hour broth culture of Pseudominas aenginoser NCIB 10421. Survivors were determined after a 7 day contact period in each week. A control contained no bactericide but was otherwise identical to the solutions on assessment.

Table 4

| Biocide | Level (ppm active ingredient) | Survivors (cells/ml) after Inoculation 1 | Inoculation 2 | Inoculation 3 |
|---|---|---|---|---|
| BBMND | 200 | <10 | <10 | <10 |
| | 100 | <10 | <10 | <10 |
| | 50 | <10 | <10 | <10 |
| | 25 | <10 | $7.3 \times 10^3$ | $>3.0 \times 10^5$ |
| BIT | 200 | <10 | <10 | <10 |
| | 100 | <10 | <10 | <10 |
| | 50 | $7.0 \times 10^3$ | $>3.0 \times 10^5$ | $>3.0 \times 10^5$ |
| | 25 | $1.9 \times 10^4$ | $>3.0 \times 10^5$ | $>3.0 \times 10^5$ |
| Control | | $2.4 \times 10^6$ | $1.15 \times 10^6$ | $1.8 \times 10^6$ |

The results indicate that BBMND has significant antibacterial activity in an oil-in-water emulsion suitable for use as a metal working fluid.

EXAMPLE 6

Antibacterial Activity of BBMND in Metal Working Fluids based on Century IBRG and Shell Dromus B Oils

BBMND was added to 25g quantities of Shell Dromus B and Century IBRG mineral oils and Century S156 Synthetic Fluid, to give active ingredient levels of 0.8, 0.4, 0.2 and 0.1%. Biocide-free controls were included. 1,2-Benzisothiazolin-3-one (BIT) in the form of "Proxel" GLX was used as a reference standard. ("Proxel" is a trademark of Imperial Chemical Industries PLC)

Variants were stored in sealed glass bottles at 50°C for 24 hours, after which 5ml volumes were transferred to 95ml deionised water in 250ml flasks.

Mineral oil emulsion variants were transferred to the orbital shaker (100 rev/min, 30°C) and were inoculated with 1ml of a 1 in 10,000 dilution (in deionised water) of an 18hr broth culture of Pseudomonas aeruginosa NCIB 10421, and after a 7 day incubation period each variant was re-inoculated with 1ml of undiluted broth culture of NCIB 10421. Survivors were determined after a 7 day contact period following each inoculum.

The above mentioned microbial challenge tests were repeated using mineral oils which had been stored at 50°C for 2 weeks.

| Oil | Biocide | Level(%) in oil | Survivors(Cells/ml in emulsions After 24 hours at 50°C Inoculation 1 Day 7 | Inoculation 2 Day 7 |
|---|---|---|---|---|
| Century IBRG | SC100424 | 0.1 | < 10 | > $3.10^5$ |
| | | 0.2 | < 10 | < 10 |
| | | 0.4 | < 10 | < 10 |
| | | 0.8 | < 10 | < 10 |
| | BIT(GXL) | 0.1 | $5.1\ 10^3$ | > $3.10^5$ |
| | | 0.2 | < 10 | > $3.10^5$ |
| | | 0.4 | < 10 | $3.6\ 10^4$ |
| | | 0.8 | < 10 | < 10 |
| | CONTROL | | $3.7\ 10^6$ | > $1.0\ 10^8$ |
| Dromus B | SC100424 | 0.1 | < 10 | < 10 |
| | | 0.2 | < 10 | < 10 |
| | | 0.4 | < 10 | < 10 |
| | | 0.8 | < 10 | < 10 |
| | BIT | 0.1 | < 10 | > $3.10^5$ |
| | | 0.2 | < 10 | > $3.10^5$ |
| | | 0.4 | < 10 | > $3.10^5$ |
| | | 0.8 | < 10 | > $3.10^5$ |
| | CONTROL | | $8.5\ 10^3$ | $6.7\ 10^7$ |
| Century IBRG | SC100424 | 0.1 | < 10 | > $3.10^5$ |
| | | 0.2 | < 10 | < 10 |
| | | 0.4 | < 10 | < 10 |
| | | 0.8 | < 10 | < 10 |
| | BIT (GXL) | 0.1 | < 10 | > $3.10^5$ |
| | | 0.2 | < 10 | < 10 |
| | | 0.4 | < 10 | < 10 |
| | | 0.8 | < 10 | < 10 |
| | CONTROL | | $3.9\ 10^6$ | > $3.10^7$ |

| | | | Survivors(Cells/ml) in emulsions After 24 hours at 50°C | |
|---|---|---|---|---|
| Oil | Biocide | Level(%) in oil | Inoculation 1 Day 7 | Inoculation 2 Day 7 |
| Dromus B | SC100424 | 0.1 | 3.6 $10^4$ | > 3.$10^3$ |
| | | 0.2 | 1.8 $10^2$ | 6.7 $10^3$ |
| | | 0.4 | < 10 | 5.9 $10^3$ |
| | | 0.8 | < 10 | < 10 |
| | BIT (GXL) | 0.1 | 5.8 $10^4$ | 6.7 $10^5$ |
| | | 0.2 | 7.6 $10^3$ | 1.4 $10^4$ |
| | | 0.4 | 2.1 $10^2$ | 5.6 $10^3$ |
| | | 0.8 | 7.0 10 | 1.0 $10^2$ |
| | CONTROL | | 3.6 $10^5$ | 6.6 $10^5$ |

The results indicate that BBMND has significant antibacterial activity following storage in Century IBRG and Shell Dromus B emulsifiable oils.

## EXAMPLE 7

Antifungal Activity of BBMND in a Metal Working Fluid Based on Century S156 Synthetic Fluid.

---

The test chemical was added to 25g quantities of Century S156 Synthetic Fluid to give active ingredient levels of 0.8, 0.4, 0.2 and 0.1%. A biocide-free control was included. 1,2-Benzisothiazolin-3-one (BIT) as "Proxel" GXL was used as a standard.

The variants were stored in sealed glass bottles at 50°C for 24 hours after which 5ml volumes were transferred to 95ml deonised water in 250ml flasks and the diluted fluids were inoculated with 1ml at a mixed fungal inoculum prepared by homogenizing a synthetic fluid, heavily contaminated with mycelial growth.

The variants were incubated statically at 25°C, and after incubation periods of one and two weeks they were examined for visible fungal growth.

| Century S156 | | Visible Fungal Growth* after | |
|---|---|---|---|
| Biocide | Level(%) in oil | 1-week incubtn. | 2-week incubtn. |
| SC 100424 | 0.1 | ++ | +++ |
| | 0.2 | ++ | +++ |
| | 0.4 | - | - |
| | 0.8 | - | - |
| BIT(GXL) | 0.1 | - | + |
| | 0.2 | - | - |
| | 0.4 | - | - |
| | 0.8 | - | - |
| CONTROL | | +++ | +++ |

*

+++, visible fungal growth comparable to that of the control
++, modeste growth but less than that of the control
+, visible growth sparse/markedly less than that of the control
-, no visible growth

The results indicate that SC100424 has significant antifungal activity in diluted Century S156 synthetic fluid.

CLAIMS

1. 5-bromo-2-bromomethyl-5-nitro-1,3-dioxan

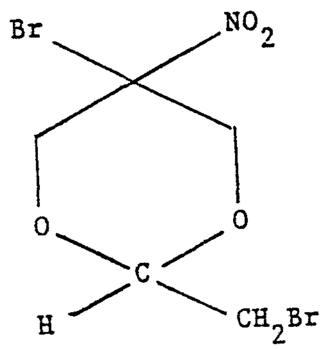

2. Use of 5-bromo-2-bromomethyl-5-nitro-1,3-dioxan as a biocide.

3. Use according to claim 2 in cooling waters, paper mill liquors, aqueous emulsions or water-based paints.

4. Use accordint to claim 2 in metal working fluids.

5. Use according to claim 3 or 4 wherein the biocide is in a concentration from 10 ppm to 1000 ppm.

6. A composition comprising a compound according to claim 1.

7. A method for the protection of a medium or substrate against attack by micro-organisms which comprises the incorporation therein of an effective amount of 5-bromo-2-bromomethyl-5-nitro-1,3-dioxan.

8. A process for the preparation of 5-bromo-2-bromomethyl 5-nitro-1,3-dioxan comprising reacting 2-bromo-2-nitro-1,3-prpoanediol with a dialkyl acetal of bromoacetaldehyde in the presence of an acid catalyst.

9. A process according to claim 8 wherein the catalyst is polyphosphoric acid.

0173531

European Patent Office

# EUROPEAN SEARCH REPORT

Application number

EP 85305885.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 4 025 533 (LAPPAS)<br>* Claim 1; abstract; column 3, lines 9-14 *<br>-- | 1,2,6, 8 | C 07 D 319/06<br>A 01 N 43/32<br>//C 10 M 173/00<br>C 10 N 40:20 |
| D,A | GB - A - 1 250 725 (HENKEL)<br>* Claims 1,2,3,7 *<br>---- | 1,2,6, 8,9 | |

TECHNICAL FIELDS SEARCHED (Int. Cl.4)

C 07 D 319/00

The present search report has been drawn up for all claims

| Place of search | Date of completion of the search | Examiner |
|---|---|---|
| VIENNA | 04-12-1985 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82